# EUROPEAN PATENT APPLICATION

(11) **EP 1 629 838 A1**
(43) Date of publication of application: **01.03.2006**
(21) Application number: 04077427.5
(22) Date of filing: 31.08.2004
(51) Int. Cl.: A61K 31/137, A61K 31/444, A61K 33/30, A61K 33/22, A61P 31/10

(54) **Footcare product with glycerin and no added water**

(71) Applicant: Beekman, R.W., 1081 AN Amsterdam (NL)
(72) Inventor: Beekman, R.W., 1081 AN Amsterdam (NL)

(57) **Abstract**

This product contains Glycerin, an Anti-Fungal product, some etheric oils and vitamines, but no added water.

It is used for the treatment of Nailmycosis and Athlete's Foot.

## Description

The invention concerns the use of Glycerin in a product for the treatment of Nailmycosis and Athlete's Foot.
It is just the combination of Glycerin with an Anti-Fungal product such as Terbinafmi Hydrochloridum, Sodium Borate, Zincpyrithione or other products, possible some etheric oils and vitamines, but no added water.

## Claims

1. Product for the treatment of Nailmycosis and Athlete's Foot containing Glycerin, some Anti-Fungalproducts, etheric oils, vitamines, but absolutely no added water.
